(19) Europäisches Patentamt
European Patent Office
Office européen des brevets

(11) Veröffentlichungsnummer: **0 185 240**
**A2**

(12) # EUROPÄISCHE PATENTANMELDUNG

(21) Anmeldenummer: 85115279.3

(22) Anmeldetag: 02.12.85

(51) Int. Cl.[4]: C 07 D 209/86

(30) Priorität: 12.12.84 DE 3445252

(43) Veröffentlichungstag der Anmeldung:
25.06.86 Patentblatt 86/26

(84) Benannte Vertragsstaaten:
CH DE FR GB LI

(71) Anmelder: BAYER AG
Konzernverwaltung RP Patentabteilung
D-5090 Leverkusen 1 Bayerwerk(DE)

(72) Erfinder: Marzolph, Gerhard, Dr.
Semmelweisstrasse 87a
D-5000 Köln 80(DE)

(72) Erfinder: Blank, Heinz Ulrich, Dr.
Am Geusfelde 35
D-5068 Odenthal(DE)

(54) **Verfahren zur Herstellung von N-substituierten Benz]c,d[indol-2-onen.**

(57) Dei Herstellung von N-substituierten Benz[c,d]indol-2-onen erfolgt durch Umsetzung von am Stickstoff nichtsubstituierten Benz [c,d]-indol-2-onen mit einem Alkylierungsmittel in einem zweiphasigen System in Gegenwart einer Base und eines Phasentransferkatalysators.

EP 0 185 240 A2

Croydon Printing Company Ltd

BAYER AKTIENGESELLSCHAFT    5090 Leverkusen, Bayerwerk
Konzernverwaltung RP
Patentabteilung    Mi/Ke-c

# Verfahren zur Herstellung von N-substituierten Benz/c,d/indol-2-onen

Die Erfindungen betrifft ein Verfahren zur Herstellung von N-substituierten Benz/c,d/indol-2-onen durch Umsetzung von am Stickstoff nichtsubstituierten Benz/c,d/-indol-2-onen mit einem Alkylierungsmittel in einem zweiphasigen System in Gegenwart einer Base und eines Phasentransferkatalysators.

N-substituierte Benz/c,d/indol-2-one können technisch hergestellt werden durch Phosgenierung von N-Alkylnaphthylaminen zu den N-Alkylnaphthylcarbamidsäurechloriden, die anschließend mit Friedel-Crafts-Katalysatoren zu den N-Alkyl-benz/c,d/indol-2-onen cyclisiert werden (z.B. GB-A-973.260). Das Verfahren hat den Nachteil, daß zur Herstellung unterschiedlicher N-Alkylderivate jeweils die entsprechenden N-Alkylnaphthylamine hergestellt werden müssen und anschließend in einer zweistufigen Synthese zu den gewünschten N-Alkyl-benz/c,d/indol-2-onen umgewandelt werden müssen.

Außerdem ist auf diesem Weg die Einführung von gegen Friedel-Crafts-Katalysatoren empfindlichen Resten nicht möglich.

In EP-A-84 853 wird ein Verfahren zur Herstellung einer Vielzahl von N-Alkylnaphtholactamen beschrieben, in dem man 1,8-Naphtholacton mit Alkyl-Aminen umsetzt. Das Verfahren hat aber den Nachteil, daß das 1,8-Naphtholacton nur in aufwendigen Syntheseschritten darstellbar ist.

In Journal Organic Chemistry USSR Vol. 2, 1972, S. 2222-25 (engl. Übers.) wird eine Arbeitsweise beschrieben, bei der Benz/c,d/indol-2-on in N-Methyl-2-pyrrolidon als Lösemittel mit Alkylbromiden oder -iodiden unter Zusatz von konzentrierte wäßriger Natronlauge in homogener Lösung alkyliert wird. Das Verfahren liefert zwar gute Ausbeuten, jedoch nur bei Verwendung der teuren Alkylbromide bzw. -iodide. Außerdem gelangt bei der wäßrigen Aufarbeitung das gesamte Lösemittel ins Abwasser, was sowohl aus ökologischer als auch aus ökonomischer Sicht unerwünscht ist.

In der DE-A 2 724 443 wird die Alkylierung von substituierten Benz/c,d/indol-2-onen mit Dialkylsulfaten und 30 %iger Natronlauge in Dimethylformamid im homogenen System beschrieben. Das Verfahren hat den Nachteil, daß nur eine Alkylgruppe des Alkylierungsmittels in die Reaktion eingeht. Außerdem geht bei der wäßrigen Aufarbeitung das gesamte Lösungsmittel verloren und die Isolierung des Produktes erfolgt aus etwa 2 %iger

Suspension. Dadurch ist das Verfahren technisch unattraktiv.

Aus Synthesis 1981, S. 1005-1008, ist bekannt, daß primäre Amide aromatischer Säuren, z.B. Benzamid, in einer siedenden Mischung aus Benzol und 50 %iger wäßriger Natronlauge in Gegenwart von Phasentransferkatalysatoren monoalkyliert werden können. Die Einführung eines zweiten Alkylrestes ist in Gegenwart wäßriger Natronlauge aber nicht möglich. Sie erfordert den Einsatz einer Mischung aus gepulvertem Natriumhydroxid und Kaliumcarbonat im Zweiphasensystem mit Benzol und Zusatz von 10 Mol-% Tetrabutylammonium-Hydrogensulfat.

Es bestand die Aufgabe, ein Verfahren zu finden, das es gestattet, aus dem gut zugänglichen Zwischenprodukt Naphthostyril eine Vielzahl von N-substituierten Benz-/c,d/indol-2-onen in wirtschaftlicher Arbeitsweise herzustellen.

Unter Alkylierung wird die Einführung von gegebenenfalls substituierten offenkettigen gesättigten und ungesättigten aliphatischen und cycloaliphatischen Kohlenwasserstoffresten, also auch die Einführung von durch Arylreste substituierten (cyclo)aliphatischen Resten verstanden, und dementsprechend wird unter Alkylierungsmittel eine Verbindung verstanden, die zu dieser Reaktion eingesetzt werden kann.

Es wurde nun gefunden, daß N-substituierte Benz/c,d/-indol-2-one der Formel

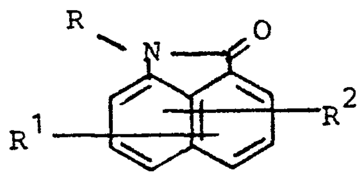

I

worin

R für einen Alkyl-, Alkenyl-, Cycloalkyl- oder Aralkylrest und

$R^1$ und $R^2$ unabhängig voneinander für Wasserstoff, Alkyl, Cycloalkyl, Aryl, Aralkyl, Cyano, Alkoxy, Aryloxy, Aralkyloxy, Acyloxy, Alkylthio, Arylthio, Aralkylthio, Alkoxycarbonyl, Aryloxycarbonyl, Alkylcarbonyl, Arylcarbonyl, Formyl, Nitro, Alkylsulfonyl, Arylsulfonyl, Aralkylsulfonyl, N,N-Dialkylcarbamoyl, N-Alkyl-N-Aryl-carbamoyl, N,N-Dialkylsulfamoyl, N-Alkyl-N-arylsulfamoyl, Alkyloxysulfonyl oder Aryloxysulfonyl stehen, oder die Reste $R^1$ und $R^2$ gemeinsam einen anellierten aromatischen oder cycloaliphatischen Rest darstellen,

durch Umsetzung eines Benz/c,d/indol-2-ons der Formel

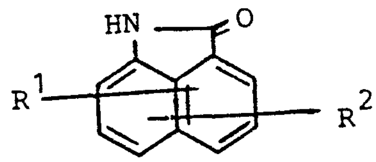

II

mit einer Verbindung der Formel

$$R - X \qquad III$$

worin X für einen als Anion $X^{(-)}$ abspaltbaren Rest steht,

in einem zweiphasigen System in Gegenwart einer Base und eines Phasentransferkatalysators der Formel

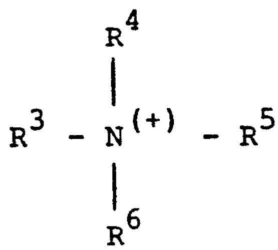

$X^{1(-)}$ IV

worin

$R^3$ - $R^6$ unabhängig voneinander Alkyl oder Aralkyl und

$X^{1(-)}$ ein Anion bedeuten,

wobei die Substituenten R und $R^1$ - $R^6$ ihrerseits nichtionische Reste tragen können,

hergestellt werden können.

Nichtionische Substituenten sind z.B.: Halogen, Alkoxy, Aryloxy, Aralkoxy, Aryl, Alkylmercapto, Arylmercapto, Aralkylmercapto, Alkylsulfonyl, Cyan, Alkoxycarbonyl, Alkylcarbonyloxy und Arylcarbonyloxy und als Substituenten der Ringe außerdem Alkyl, Aralkyl oder Nitro.

Unter Acyl wird vorzugsweise Alkylcarbonyl, Arylcarbonyl, Alkylsulfonyl, Arylsulfonyl, Alkoxycarbonyl, Aryloxycarbonyl, Alkylaminocarbonyl und Arylaminocarbonyl verstanden.

Bevorzugt stehen Alkyl für $C_1$-$C_{20}$-Alkyl, insbesondere für $C_1$-$C_4$-Alkyl, Alkenyl für $C_2$-$C_4$-Alkenyl, Cycloalkyl für Cyclohexyl, Aralkyl für Benzyl oder Phenylethyl und Aryl für Phenyl oder Naphthyl.

Halogen steht insbesondere für Fluor, Chlor oder Brom.

Bevorzugt werden nach dem neuen Verfahren N-substituierte Benz/c,d/indol-2-one der Formel

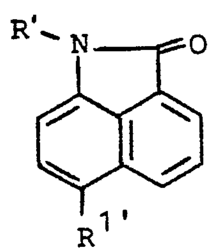

V

worin

R' $C_1$-$C_4$-Alkyl, das durch CN, Cl oder Br substituiert sein kann, Allyl, Methallyl oder Benzyl, das durch Cl, Br, $C_1$-$C_4$-Alkyl oder $C_1$-$C_4$-Alkoxy substituiert sein kann, und

$R^{1'}$ Wasserstoff, Chlor oder Brom bedeuten,

durch Umsetzung eines Benz/c,d/indol-2-ons der Formel

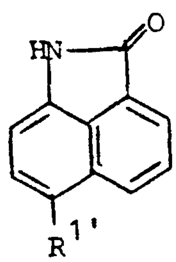

VI

mit einer Verbindung der Formel

R' - X' VII

worin

X' für einen unter den Verfahrensbedingungen als Chlorid, Bromid, Jodid, Sulfat, Methosulfat, Ethosulfat, Benzolsulfonat, Tosylat oder Mesylat abspaltbaren Rest steht,

in einem zweiphasigen System in Gegenwart einer Base und eines Phasentransferkatalysators der Formel

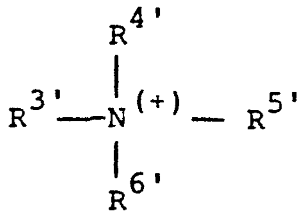 $X^{1'(-)}$ VIII

worin

$R^{3'}$ - $R^{6'}$ unabhängig voneinander $C_1$-$C_{12}$-Alkyl, Benzyl oder Methylbenzyl und

$X^{1'(-)}$ $Cl^{(-)}$, $Br^{(-)}$, $OH^{(-)}$ oder $HSO_4^{(-)}$

bedeuten,
hergestellt.

Die Benz/c,d/indol-2-one II kommen als reine Substanzen, als isolierte Rohprodukte oder als Rohlösungen aus der Cyclisierung der α-Naphthylisocyanate zum Einsatz.

Bevorzugte Verbindungen III sind Methylchlorid, Ethylchlorid, n- und iso-Propylchlorid und -bromid, n-Butylbromid, Benzylchlorid, Allylchlorid, Dimethyl- und Diethylsulfat.

Die Verbindungen III werden in einer Menge von 1 bis 3 Mol pro Mol Benz/c,d/indol-2-on II, bevorzugt in einer Menge von 1,1 bis 2,5 Mol, besonders bevorzugt in einer Menge von 1,3 bis 2,0 Mol, eingesetzt.

Die für das Verfahren verwendeten Basen sind starke anorganische Basen. Bevorzugt eingesetzt werden NaOH, KOH oder CaO. Die Basen können als Feststoffe, Lösungen oder Suspensionen eingesetzt werden.

Die Basen werden in einer Menge von 1 bis 3 Mol pro Mol Benz/c,d/indol-2-on II eingesetzt, bevorzugt 1,2 bis 2,5 Mol, besonders bevorzugt 1,3 bis 2,0 Mol pro Mol II.

Die Basen werden in der Regel equimolar zum Alkylierungsmittel III eingesetzt, sie können aber auch in stöchiometrischen Überschuß oder Unterschuß eingesetzt werden, sofern sich daraus Vorteile ergeben.

Die Verwendung von weniger als 1 Mol Alkylierungsmittel III oder Base ist zwar möglich, es ist dann aber die Abtrennung von Ausgangssubstanz II notwendig.

Es ist auch möglich Mischungen der genannten Basen einzusetzen.

Das erfindungsgemäße Verfahren wird in einem zweiphasigen System durchgeführt, das aus einer wäßrigen Phase und einem mit Wasser nicht mischbaren, unter den Reaktionsbedingungen inerten, organischen Lösungsmittel besteht.

Die wäßrige Phase besteht dabei aus einer Lösung oder Suspension der für die Reaktion verwendeten anorganischen Base. Dabei kommen 10 bis 60 %ige wäßrige Lösungen von NaOH oder KOH oder 60 bis 100 %ige Suspensionen von NaOH

oder KOH in Wasser oder Anschlämmungen von CaO in Wasser zum Einsatz. In einer bevorzugten Variante kommen 30 bis 60 %ige wäßrige Lösungen von NaOH oder KOH zum Einsatz.

In einer weiteren bevorzugten Arbeitsweise wird die Base als wasserarmer Feststoff eingesetzt, so z.B. die Natronlauge als 98 %iger Feststoff und die Kalilauge als 87 %iger Feststoff, die beide als solche im Handel sind. Die Feststoffe können dabei als Pulver, Schuppen oder Plätzchen eingesetzt werden.

Als mit Wasser nicht mischbare, unter den Reaktionsbedingungen inerte, organische Lösungsmittel kommt eine große Zahl technischer Lösungsmittel infrage.

Dies sind z.B. aromatische Kohlenwasserstoffe wie Benzol, Toluol, Xylole, Chlorbenzol, Dichlorbenzole, sowohl reine Isomere wie auch Mischungen, Trichlorbenzole, Chlortoluole oder aliphatische Chlorkohlenwasserstoffe wie Methylendichlorid, Ethylendichlorid, Trichlorpropan, Tetrachlormethan oder aliphatische oder aromatische Ether wie Diethylether, Diisopropylether, Anisol, Phenetol.

Bevorzugt eingesetzt werden aromatische Kohlenwasserstoffe wie Toluol, o-Dichlorbenzol, o/p-Dichlorbenzol-Isomerengemische, Trichlorbenzol.

Das organische Lösungsmittel kommt in einer solchen Menge zum Einsatz, daß am Beginn der Reaktion eine Lösung oder rührfähige Suspension des Naphtholactames der Formel II vorliegt. Dies ist in der Regel der Fall, wenn man mit 10 bis 70 %igen Lösungen bzw. Suspensionen arbeitet. Bevorzugt werden 30 bis 60 %ige Suspensionen eingesetzt.

Als Phasentransferkatalysatoren IV seien beispielsweise die Chloride und Bromide der folgenden Ammoniumionen genannt:

Tetramethylammonium, Tetraethylammonium, Tetrabutylammonium, Tributylethylammonium, Triethylbenzylammonium, Tributylbenzylammonium, Trioctylmethylammonium, Trimethyldodecylammonium und Dimethylcetylbenzylammonium.

Die Phasentransferkatalysatoren IV kommen in einer Menge von 0,001 bis 0,1 Mol, bevorzugt von 0,005 bis 0,05, besonders bevorzugt von 0,01 bis 0,03 Mol pro Mol Naphthostyril zum Einsatz.

In einer bevorzugten Variante wird der Katalysator im Reaktionsgemisch aus einem Amin der Struktur

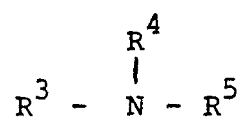

V

und der Verbindung der Struktur

$$R - X \qquad III$$

hergestellt. Dabei hat R die Bedeutung von $R^6$ und die Reste $R^3$, $R^4$ und $R^5$ haben die oben dargelegte Bedeutung. Dabei kommt das Amin V in einer Menge von 0,01 bis 1 Mol zum Einsatz.

Die Reaktion wird bei einer Temperatur von 20 bis 200°C, bevorzugt bei 30 bis 150°C, besonders bevorzugt bei 50 bis 120°C, durchgeführt.

Die Reaktion wird drucklos oder unter Druck durchgeführt, wobei sich die Anwendung von Druck nach den Siedepunkten von Lösungsmittel und Agens III richtet.

Die Reaktionszeit richtet sich nach der angewendeten Temperatur und der Art und Menge des angewendeten Katalysators und beträgt in der Regel 0,5 bis 10 Stunden.

Nach Durchführung der Reaktion wird die organische Phase durch Filtration, Extraktion oder Phasentrennung von anorganischen Rückständen oder der Wasserphase getrennt, gegebenenfalls geklärt und eingedampft.

Das Rohprodukt kann durch Kristallisation oder Destillation gereinigt werden.

Die N-substituierten Benz/c,d/indol-2-one sind bekannte Zwischenprodukte zur Herstellung von Farbstoffen, die beispielsweise in der GB-A-973 259 beschrieben werden.

Die im folgenden gezeigten Beispiele sollen das Verfahren erläutern.

Beispiel 1

In 195 g o-Dichlorbenzol werden 225,3 g 75 %iges rohes Naphthostyril (1 Mol) suspendiert. Dann gibt man 177,7 g (2 Mol) 45 %ige NaOH und 10 g Tetrabutylammoniumbromid zu, heizt auf 80°C und drückt 129 g Ethylchlorid auf. Man rührt 2 h bei 80°C nach, kühlt und entspannt. Man gibt 300 ml Wasser zu, verrührt 10 Minuten mit 10 g Celite und saugt ab. Das Celite wird mit 50 ml o-Dichlorbenzol nachgewaschen. Die Phasen werden getrennt, die organische Phase einmal mit 50 ml Wasser ausgeschüttelt und das o-Dichlorbenzol bei 20 mbar abdestilliert. Das rohe Ethylnaphtholactam wird bei 180-210°C Kopftemperatur und 5-6 mbar destilliert. Man erhält 188 g 95,5 %iges Ethylnaphtholactam mit Fp.: 65-67°C.
Ausbeute: 91 % der Theorie.

Beispiel 2

177,9 g 95 %iges Naphthostyril (1 Mol) werden in 200 g Toluol suspendiert. Dazu gibt man 61,2 g 98 %iges NaOH-Plätzchen und 8 g Tetrabutylammoniumchlorid. Die Mischung wird mit 129 g (2 Mol) Ethylchlorid versetzt und 1,5 h bei 100°C gerührt. Der kalte Autoklaveninhalt wird mit 300 g Wasser versetzt und die Wasserphase abgetrennt. Die organische Phase wird im Vakuum destilliert. Man erhält 182,7 g 97 %iges N-Ethylnaphtholactam.
Ausbeute: 90 % der Theorie.

Beispiel 3

177,9 g 95 %iges Naphthostyril (1 Mol) werden in 200 g o-Dichlorbenzol suspendiert, mit 177,7 g (2 Mol) 45 %iger NaOH und 10 g Tributylbenzylammoniumchlorid versetzt und dann 129 g Ethylchlorid aufgedrückt. Man rührt 10 h bei 60°C und entspannt. Der Autoklaveninhalt wird durch Absaugen von ausgefallenen Salzen befreit, die organische Plase abgetrennt und einmal mit Wasser gewaschen. Nach Abdestillieren des o-Dichlorbenzols wird das Produkt bei 190-220°C/6-9 mbar destilliert. Man erhält 174,4 g 96 %iges Ethylnaphtholactam.
Ausbeute: 85 % der Theorie.

Beispiel 4

Von 815 g einer 17 %igen Lösung von Naphthostyril in o-Dichlorbenzol aus einem Cyclisierungsansatz von α-Naphthylisocyanat werden 470 g o-Dichlorbenzol im Vakuum abdestilliert. Die verbleibende Suspension enthält 138,6 g (0,82 Mol) Naphthostyril und wird mit 177,7 g (2 Mol) 45 %iger NaOH und 10 g Trioctylmethylammoniumchlorid versetzt. Dann werden 129 g (2 Mol) Ethylchlorid aufgedrückt und der Ansatz 6 h bei 70°C gerührt. Nach Entspannen werden 350 g Wasser zugegeben, die organische Phase abgetrennt und destilliert. Man erhält 146,5 g 97 %iges N-Ethylnaphtholactam.
Ausbeute: 88 % der Theorie.

Beispiel 5

In der Suspension aus 229 g 86 %igem Naphthostyril in 200 g Dichlorbenzol-Isomerengemisch gibt man 10 g Tributylamin und 129 g Ethylchlorid. Man heizt die Mischung 1 h auf 120°C, kühlt auf 80°C ab und pumpt in 1 h 177,7 g (2 Mol) 45 %ige NaOH zu. Man rührt noch 3 h bei 80°C nach, entspannt und verrührt mit 350 g Wasser. Die organische PHase wird abgetrennt und destilliert. Man erhält 184,4 g 94 %iges Ethylnaphtholactam.
Ausbeute: 88 % der Theorie.

In diesem Beispiel wird vor der Reaktion Ethyltributylammoniumchlorid als Phasentransferkatalysator gebildet.

Beispiel 6-10

Analog zu Beispiel 2 werden statt Ethylchlorid die folgenden Alkylierungsmittel eingesetzt, so erhält man die entsprechenden Benz/c,d/indol-2-one in den angegebenen Ausbeuten.

| Beispiel | Alkylierungs-mittel | Ausbeute (%) | Fp (°C) | Kp (°C/mbar) |
|---|---|---|---|---|
| 6 | Methylchlorid | 91 | 78 | |
| 7 | n-Propylchlorid | 86 | | 150-155/0,5 |
| 8 | n-Butylbromid | 88 | | 172-176/0,5 |
| 9 | Diethylsulfat | 92 | 65-66 | |
| 10 | Dimethylsulfat | 89 | 76-78 | |

Beispiel 11

177,9 g 95 %iges Naphthostyril (1 Mol) werden in 150 g Chlorbenzol suspendiert, mit 177,7 g (2 Mol) 45 %ige NaOH und 10 g Tetrabutylammoniumbromid versetzt. Man erhitzt auf 80°C und tropft in 1 h 190 g (1,5 Mol) Benzylchlorid zu und rührt noch 5 h bei 80°C nach. Dann gibt man 350 ml Wasser zu, trennt die organische Phase ab und destilliert das Chlorbenzol im Vakuum ab. Der Rückstand wird aus Ethanol umkristallisiert. Man erhält 229 g 96 %iges N-Benzylnaphtholactam mit Fp.: 118°C.
Ausbeute: 85 % der Theorie.

0185240

Patentansprüche

1. Verfahren zur Herstellung von N-substituierten Benz/c̄,d̲7indol-2-onen durch Alkylierung von am Stickstoff unsubstituierten Benz/c̄,d̲7indol-2-onen, dadurch gekennzeichnet, daß das Verfahren in einem zweiphasigen System in Gegenwart einer Base und eines Phasentransferkatalysators durchgeführt wird.

2. Verfahren nach Anspruch 1 zur Herstellung von N-substituierten Benz/c̄,d̲7indol-2-onen der Formel

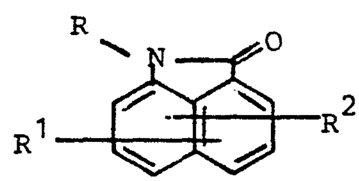

worin

R für einen Alkyl-, Alkenyl-, Cycloalkyl- oder Aralkylrest und

$R^1$ und $R^2$ unabhängig voneinander für Wasserstoff, Alkyl, Cycloalkyl, Aryl, Aralkyl, Cyano, Alkoxy, Aryloxy, Aralkyloxy, Acyloxy, Alkylthio, Arylthio, Aralkylthio, Alkoxycarbonyl, Aryloxycarbonyl, Alkylcarbonyl, Arylcarbonyl, Formyl, Nitro, Alkylsulfonyl, Arylsulfonyl, Aralkylsulfonyl, N,N-Dialkylcarbamoyl, N-Alkyl-N-Arylcarbamoyl, N,N-Dialkylsulfamoyl, N-Alkyl-N-arylsulfamoyl, Alkyloxysulfonyl oder Aryloxysulfonyl stehen oder

die Reste $R^1$ und $R^2$ gemeinsam einen anellierten aromatischen oder cycloaliphatischen Rest darstellen,

durch Umsetzung von Benz/c,d/indol-2-onen der Formel

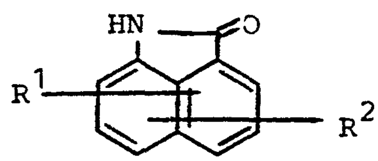

mit Alkylierungsmitteln der Formel

R - X

worin X für einen als Anion $X^{(-)}$ abspaltbaren Rest steht, und die Substituenten R, $R^1$ und $R^2$ ihrerseits nichtionische Reste tragen können, dadurch gekennzeichnet, daß das Verfahren in einem zweiphasigen System in Gegenwart einer Base und eines Phasentransferkatalysators der Formel

0185240

$$\begin{array}{ccccc} & & R^4 & & \\ & & | & & \\ R^3 & - & N^{(+)} & - & R^5 \qquad X^{1(-)} \\ & & | & & \\ & & R^6 & & \end{array}$$

worin

$R^3$ - $R^6$ unabhängig voneinander Alkyl oder Aralkyl und

$X^{1(-)}$ ein Anion bedeuten, und

die Substituenten $R^3$-$R^6$ ihrerseits nichtionische Reste tragen können, durchgeführt wird.

3. Verfahren nach Anspruch 1 zur Herstellung von N-substituierten Benz[c,d]indol-2-onen der Formel

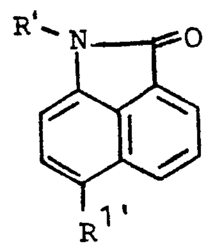

worin

R' $C_1$-$C_4$-Alkyl, das durch CN, Cl oder Br substituiert sein kann, Allyl, Methallyl oder Benzyl, das durch Cl, Br, $C_1$-$C_4$-Alkyl oder $C_1$-$C_4$Alkoxy substituiert sein kann, und

$R^1$ Wasserstoff, Chlor oder Brom bedeuten,

durch Umsetzung von Benz/c,d/indol-2-onen der Formel

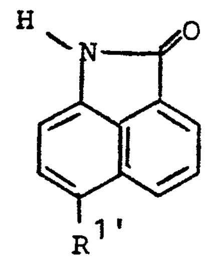

mit Alkylierungsmitteln der Formel

$$R' - X'$$

worin

X' für einen unter den Verfahrensbedingungen als Chlorid, Bromid, Jodid, Sulfat, Methosulfat, Ethosulfat, Benzolsulfonat, Tosylat oder Mesylat abspaltbaren Rest steht,

dadurch gekennzeichnet, daß das Verfahren in einem zweiphasigen System in Gegenwart einer Base und eines Phasentransferkatalysators der Formel

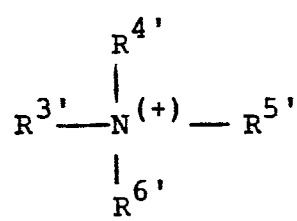 $X^{1'(-)}$

worin

$R^{3'}-R^{6'}$ unabhängig voneinander $C_1$-$C_{12}$-Alkyl, Benzyl oder Methylbenzyl und

$X^{1'(-)}$ $Cl^{(-)}$, $Br^{(-)}$, $OH^{(-)}$ oder $HSO_4^{(-)}$ bedeuten,

durchgeführt wird.

4. Verfahren nach Anspruch 1, dadurch gekennzeichnet, daß als Alkylierungsmittel Methylchlorid, Ethylchlorid, n-Propylchlorid, Isopropylbromid, n-Butylbromid, Allylchlorid oder Benzylchlorid in einer Menge von 1-3 Mol pro Mol Benz/c,d/indol-2-on eingesetzt wird.

5. Verfahren nach Anspruch 1, dadurch gekennzeichnet, daß als Base eine starke anorganische Base in einer Menge von 1 - 3 Mol pro Mol Benz/c,d/indol-2-on eingesetzt wird.

6. Verfahren nach Anspruch 1, dadurch gekennzeichnet, daß als Phasentransferkatalysator das Chlorid oder Bromid der Kationen, Tetramethylammonium, Tetraethylammonium, Tetrabutylammonium, Tributylethylammonium, Triethylbenzylammonium, Tributylbenzylammonium, Trioctylmethylammonium, Trimethyldodecylammonium und Dimethylcetylbenzylammonium in einer Menge von 0,001 - 0,1 Mol pro Mol Benz/c,d/indol-2-on eingesetzt wird.

7. Verfahren nach Anspruch 1, dadurch gekennzeichnet, daß der Phasentransferkatalysator im Reaktionsgemisch aus einem Amin der Formel

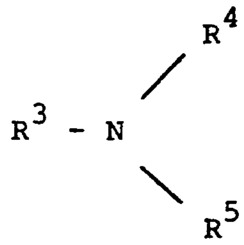

und dem Alkylierungsmittel gebildet wird.

8. Verfahren nach Anspruch 1, dadurch gekennzeichnet, daß das zweiphasige System aus einer wäßrigen Phase und einem mit Wasser nicht mischbaren, unter den Reaktionsbedingungen inerten organischen Lösungsmittel besteht.

9. Verfahren nach Anspruch 1, dadurch gekennzeichnet, daß die wäßrige Phase aus einer 10-60 %igen wäßrigen Lösung oder 60-100 %iger wäßrigen Suspension der Base oder einer wasserarmen festen anorganischen Base besteht, und als organisches inertes Lösungsmittel Toluol, Xylol, Dichlorbenzol, Chlorbenzol, Trichlorbenzol, Chlortoluol, Methylendichlorid, Ethylendichlorid oder Trichlorpropan verwendet wird.

10. Verfahren nach Anspruch 1, dadurch gekennzeichnet, daß die Reaktion bei einer Temperatur von 20-200°C durchgeführt wird.